(19) Europäisches Patentamt / European Patent Office / Office européen des brevets

(11) **EP 2 827 145 B1**

(12) **EUROPEAN PATENT SPECIFICATION**

(45) Date of publication and mention
of the grant of the patent:
**08.04.2020  Bulletin 2020/15**

(51) Int Cl.:
*G01N 33/00* *(2006.01)*    *G01D 18/00* *(2006.01)*

(21) Application number: **14003449.7**

(22) Date of filing: **08.10.2014**

(54) **Sensor calibration**

Sensorkalibrierung

Étalonnage d'un capteur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.01.2015  Bulletin 2015/04**

(73) Proprietor: **Sensirion AG**
**8712 Stäfa (CH)**

(72) Inventors:
• **Schanz, Christoph**
  **CH-8712 Stäfa (CH)**
• **Poulsen, Eva**
  **CH-8712 Stäfa (CH)**
• **Gerner, Pascal**
  **CH-8712 Stäfa (CH)**

• **Graf, Markus**
  **CH-8712 Stäfa (CH)**

(74) Representative: **Toleti, Martin**
**E.Blum & Co. AG**
**Vorderberg 11**
**8044 Zürich (CH)**

(56) References cited:
**EP-A1- 1 628 132    US-B2- 7 291 507**

• **MICHAEL DEEDS ET AL: "Carrier-Level Packaging and Reliability of a MEMS-Based Safety and Arming Device", MRS PROCEEDINGS, vol. 682E, N.4.4, 31 December 2001 (2001-12-31), pages 1-12, XP055158511, DOI: 10.1557/PROC-682-N4.4**

EP 2 827 145 B1

**Description**

Technical Field

The invention relates to a method for calibrating a sensor chip, and to a production line for manufacturing a calibrated sensor chip.

Background Art

Sensors that are integrated on semiconductor chips offer a whole new range of applications in view of their small scale.

One known type of a humidity sensor that uses a layer of a humidity sensitive material arranged on a semiconductor chip is described in WO 01/42776. Another type of sensor e.g. uses metal oxide material and is adapted to measure various types of substances in gases or liquids, such as Ethanol, Sulfide, CO, $CO_2$, $NO_x$.

Semiconductor chips are usually manufactured in wafers, where each wafer may comprise hundreds or more chips. EP 1 628 132 A1 suggests to calibrate semiconductor chips comprising sensing elements, which are referred to as sensor chips in the following, on the wafer. This allows to calibrate a large number of sensor chips quickly and to eliminate those sensors that cannot be calibrated from the further manufacturing steps. Furthermore, it requires only a small volume of calibration fluid for calibrating a large number of sensor chips, in case the sensor chips are sensitive to a substance in a fluid.

Packaging of MEMS devices is addressed in document Michael Deeds et al: "Carrier-level Packaging and Reliability of a MEMS-Based Safety and Arming Device", MRS Proceedings, vol 682E, 31 December 2001, article number N4.4, pages 1-12. The document addresses the architecture of the device, wherein the impact of moisture on packaged MEMS systems and test structures is investigated.

However, it was found by the applicant that manufacturing steps performed subsequent to the calibration may impact the measuring characteristic of the sensor chip, e.g. owed to stress induced by such manufacturing steps.

Disclosure of the Invention

Hence, it is desired to improve the measuring results of a sensor chip.

This problem is solved by a method for calibrating a sensor chip. According to this method a first calibration measurement is performed with the sensor chip. As a result of this calibration measurement, first calibration data is determined. After the first calibration measurement, a manufacturing step is applied to the sensor chip. This manufacturing step may include, for example, packaging the sensor chip, e.g. one or more of attaching a cap to the sensor chip, casting a mould compound to / around the sensor chip, arranging the sensor chip in a housing, etc. Other manufacturing steps that may be applied may include one or more of applying a layer to the sensor chip, attaching contact elements, in particular solder bumps to the sensor chip, applying a membrane to a packaged sensor chip, attaching, in particular soldering the sensor chip to a circuit board or a leadframe, or separating the sensor chip from a wafer, etc. What is common to these manufacturing steps is that a sensing element or an electronic circuitry of the sensor chip is mechanically and/or chemically impacted during this manufacturing step, in particular in form of stress, and as such impact future measurement results.

Hence, it is envisaged to perform a further calibration measurement with the sensor chip after the one or more manufacturing steps. As a result of this further calibration measurement, further calibration data is determined. Finally, at least one calibration parameter is determined as a function of both said first calibration data as well as said further calibration data, and this at least one calibration parameter is stored in a memory of the sensor chip.

It is preferred not to store any calibration parameter derived only from the first calibration data to the sensor chip, but only the calibration parameter(s) obtained from both calibration measurements. The first calibration data, or any data derived therefrom, preferably is at least temporarily stored in a storage of a production line (i.e. outside the sensor chip) for manufacturing calibrated sensor chips. The at least one calibration parameter is determined dependent at least on the first and the further calibration data. Hence, the at least one calibration parameter takes into account an impact of the manufacturing step in between the at least two calibration measurements such that any stress etc. induced is compensated for by determining the calibration parameter(s) as a function of the first and further calibration data and having this calibration parameter(s) applied to the signal sensed by a sensing element of the sensor chip during operation.

Preferably, prior to the manufacturing step, the sensor chip yet is part of a wafer for manufacturing multiple sensor chips from. Hence, the first calibration measurement may be performed on the wafer, while in the present example, the further calibration measurement may be performed on the individual sensor chip after having it has been separated from the wafer. Hence, in one embodiment, the manufacturing step may specifically include the dicing of the sensor chip from the wafer, which may require an adjustment of the earlier first calibration data. As a result, an efficient wafer lever calibration can be performed in combination with achieving a precise calibration in view of effects originating from the manufacturing step being compensated for by the result of a second calibration measurement.

Advantageously, in the first calibration measurement, a larger set of calibration data is obtained than in the further calibration measurement. In particular, a larger number of measured values is obtained in the first calibration measurement than in the further calibration measurement. This is particularly time-saving if the first calibration measurement can e.g. be carried out on the wafer (or in another arrangement where a large number of sensor chips can be exposed to the given reference conditions in parallel), while the second calibration measurement is carried out by exposing a smaller number of sensor chips in parallel to the reference conditions, e.g. by exposing individual sensor chips to these reference conditions.

It is preferred that in a calibration measurement, be it the first one, a second one, or the further one, the sensor chip is exposed to a number of defined reference conditions. If the sensor is a gas sensor sensitive to a certain gas component, e.g. humidity or an organic substance, the sensor is exposed to various known levels of this gas component and a measurement is carried out at each level, and (since this type of sensor often is also sensitive to environmental temperature) this measurement is advantageously repeated for differing temperatures. Similarly, the measurement can e.g. be repeated for differing supply voltages if the sensor is sensitive to the exact value of the supply voltage.

In other embodiments, the sensor chip may contain a sensing element that is sensitive to another property of the environment such as temperature, pressure, flow, etc. Hence, the sensor chip may in this instance represent a temperature sensor, a pressure sensor or a flow sensor. In this case, in a calibration measurement, the sensor chip is exposed to environments with defined magnitudes of the property to be measured.

In other words, in a calibration measurement, the sensor chip is exposed to a number of differing reference conditions. These reference conditions typically include different values of the variable that the sensor chip is supposed to measure (such as humidity or pressure), but they may also include different values of other variables that the sensor chip is not necessarily supposed to measure, but that its properties depend on, such as temperature and supply voltage.

In more mathematical terms, the sensor chip is supposed to generate at least one signal y as

$$y = F(x1, \ldots xN, p1, \ldots pM), \qquad (1)$$

with

- x1 ... xN (N >= 1) being input signals that the sensor chip is sensitive to and that it is able to detect, and
- p1 ... pM being the calibration parameters.

F is a function that can e.g. be implemented as a lookup table, a mathematical function, or any other algorithm that can be executed by the sensor chip.

The input signals x1 ... xN typically include the signals that are electronically measured on a sensing area of the chip. In particular, the input signals include signals that are strongly dependent on the environmental variable(s) that the sensor chip is supposed to measure, such as a value depending on the dielectric constant of a polymer used in a humidity sensor. The input signals may, however, also include further values that are dependent on other variables (including operating parameters) that the sensor chip is sensitive to, such as a value derived from the current temperature of the sensor chip and/or from the current supply voltage applied to the sensor chip.

The calibration parameters can e.g. be the values stored in a N-dimensional lookup table. In this case, the sensor chip is structured and adapted to look up the value y in this lookup table, optionally using interpolation between the closest values stored in the lookup table.

In a simple embodiment, for a temperature sensor chip, function F may e.g. be expressed as a simple linear relationship of the type

$$y = p1 + p2 \cdot x1, \qquad (2)$$

in which case N = 1 and M = 2, and x1 is a measured voltage that depends on the sensor temperature, while y is the output value that the sensor chip is specified to deliver at this temperature. The first calibration measurement may e.g. include exposing the sensor chip to a large number of different reference temperatures, each of which gives rise to a different value of x1. This allows to determine first calibration parameters p1', p2' using linear regression analysis. These first calibration parameters p1', p2', or the whole calibration data derived in the first calibration measurement, is stored advantageously outside the sensor chip.

The manufacturing step, which is performed after the first calibration measurement, may affect the sensor chip, and in particular its calibration parameters. For example, the manufacturer of the sensor chip may find that the offset parameter p1 typically changes by a few percent during the manufacturing step, while the slope parameter p2 remains mostly unaffected. Hence, a single measurement, or a very small number of measurements, may suffice in the further calibration step to determine the new value of offset parameter p1, while slope parameter p2 can be assumed to be equal to parameter p2' measured in the first calibration step. The final calibration parameters p1, p2 determined in this way can then be stored in the sensor chip for being used during its normal operation.

The final calibration parameter(s) can be derived from the first and the further calibration data in different ways:

- As in the example described above, the first calibration data can be used to determine first calibration parameters p1', p2,' ..., some of which may then be replaced or corrected in view of the second calibration data.
- Alternatively, the first and second calibration data can be directly combined for calculating the final calibration parameter(s). For example, the first and second calibration data can both be used in a linear or non-linear regression analysis for calculating the calibration parameter(s). In this regression analysis, the sum of a measure of the deviations (e.g. expressed as the squares of the differences) between the calculated and expected values is minimized, in which sum the measurements of the second calibration data can be given stronger weights than those of the first calibration data. Algorithms for regression analysis minimizing a sum of weighted deviations are known to the skilled person.

The scope of the present invention is not limited to exactly two calibration measurements. There may be three or more calibration measurements with manufacturing steps in between. In a preferred embodiment, a second calibration measurement is performed with the sensor after having performed the manufacturing step and second calibration data is determined as a result thereof. After the second calibration measurement, the sensor chip may be exposed to a further manufacturing step. The further calibration measurement in turn may be performed after the further manufacturing step. The final calibration data is then determined from the first, the second and the further calibration data.

Specifically, the first calibration measurement may be carried out on a wafer comprising the sensor chip, and preferably multiple other sensor chips, yet without any packaging. Then, the wafer is exposed to further wafer-level manufacturing steps, such as steps for coating the sensor chips or steps for applying further structures in the wafer. After this manufacturing step a second calibration measurement is applied and second calibration data is determined. The second calibration data may be stored in a storage of the production line similar to the first calibration data.

After the second calibration measurement, one or more further manufacturing steps may be performed, such as one or more of applying a wafer scale package to the wafer in view of the sensor chip yet being part of the wafer. In one of these further manufacturing steps, the wafer may be diced into the individual sensor chips, and the further calibration measurement may be performed after dicing, i.e. with the individual sensor chip.

According to another aspect of the present invention, a production line is introduced for manufacturing a calibrated sensor chip according to any embodiment of the method as previously introduced. The production line comprises a first apparatus for performing the first calibration measurement, such as a prober, in particular a prober suited for carrying out measurements on a wafer-level. At a work station of the production line, the manufacturing step may be performed, while the further calibration measurement is performed either at the first apparatus or at a dedicated further apparatus. A computing device is provided for determining the final calibration parameter(s).

Other advantageous embodiments are listed in the dependent claims as well as in the description below.

Brief Description of the Drawings

Embodiments of the invention are introduced in the following detailed description. Such description makes reference to the annexed drawings, wherein:

Fig. 1 illustrates a schematic production line according to an embodiment of the present invention, thereby illustrating a method according to an embodiment of the present invention,
Fig. 2 illustrates a schematic production line according to another embodiment of the present invention, thereby illustrating a method according to another embodiment of the present invention,
Fig. 3 shows a schematic view of an apparatus for calibrating a sensor chip as used in a method and a production line according to embodiments of the present invention, and
Fig. 4. illustrates a sample sensor chip in a longitudinal cut to be calibrated by a method or a production line according to embodiments of the present invention.

Detailed Description of the Drawings

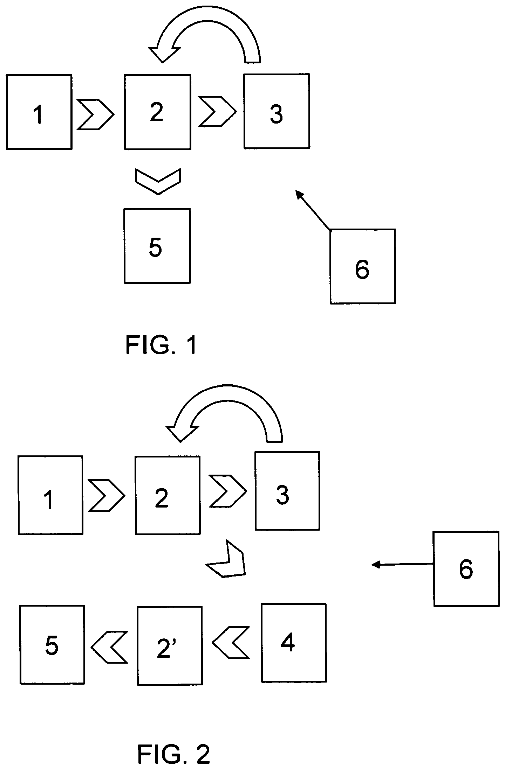

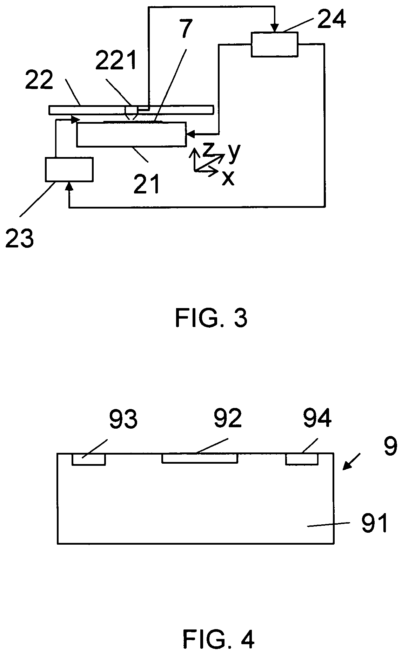

Figure 1 illustrates a schematic production line according to an embodiment of the present invention. A sensor chip that is to be calibrated and is continued to be manufactured in the production line is illustrated in Figure 4 in a schematic longitudinal cut: The sensor chip 9 comprises a semiconductor substrate 91, such as a silicon substrate. A sensing element 92 is arranged on a front side of the substrate 91. An electronic circuit 93 is integrated into the substrate 91 and preprocesses signals supplied by the sensing element 92. In addition, an on-chip memory 94 is provided for storing final calibration parameters.

Electronic circuit 93 is adapted and structured to process the measured signals using the calibration parameters stored in memory 94, e.g. by applying an calibration function of the general type of Eq. (1) or the specific type of Eq. (2), as described above.

A sensor chip such as illustrated in Figure 4 may be supplied by a work station 1 of the production line according to Figure 1 for further processing. There may be multiple previous manufacturing steps, such as manufacturing the electronic circuit 93 and the memory 94 by CMOS-processing, applying the sensing element, etc. At this stage, the sensor chip may not be separated yet from the wafer. Accordingly, the wafer preferably contains a two-dimensional matrix of sensor chips that are basically ready for operation but that still need to be calibrated, cut and, where applicable, packaged and / or diced. Hence, such a wafer is supplied to an apparatus 2 for calibrating the and preferably all sensor chips on the wafer.

The apparatus 2 - which is also referred to as prober - performs a first calibration measurement on the wafer. Figure 3 schematically illustrates such an apparatus 2 for calibrating a sensor chip. The apparatus comprises a chuck 21 and a lid 22 that are movable relative to each other. The lid 22 comprises a probe head 221. A wafer 7 is placed on the chuck 21 underneath the lid 22 and is electrically contacted by electrodes of the probe head 221.

An apparatus of this type is e.g. described in EP 1628132.

In one example, the sensor chip represents a humidity sensor. A humidity generator 23 of the apparatus 2 may be controlled by a control unit 24 of the apparatus 2 to generate a gas with a known humidity level. The gas may e.g. be air or nitrogen. A pump (not shown) in the humidity generator 23 then feeds the humid gas through a tube, from which it enters the vicinity of the wafer 7 as is indicated by the arrow. In response to the exposure of the sensor chips to the humid gas, the sensor chips supply a measurement signal which is received by the electrodes of the probe head 221. The calibration data determined in this way, or data derived therefrom, is stored in a computing device of the control unit 24, or of the apparatus or of the production line. The probe head 221 may be configured to contact many sensor chips of the wafer 7 simultaneously. In an alternative, only one or a few sensor chips are contacted simultaneously by the electrodes, and the control unit 24 may control an actuator of either the chuck 21 or the lid 22 to position the various sensor chips on the wafer 7 in sequence under the probe head 221 for taking the first calibration measurement.

In another embodiment, the sensing element 92 of the sensor chip 9 is sensitive to temperature. Then no humidity generator 23 is required but only a heater or cooler for heating and/or cooling the wafer 7 or an environment of the wafer 7 during the calibration measurement. In particular, the lid 2 and / or the chuck 21 may be temperature controlled to generate a highly homogeneous temperature distribution in a gap there between, in particular if lid 22 and chuck 21 are kept at the same temperature.

The data from the first calibration measurement is stored outside the sensor chip together with a unique identifier of the sensor chip it belongs to (unique at least in the sense that the sensor chip can be uniquely identified in a manufacturing batch). Such an identifier can e.g. be derived from an identifier of the wafer in addition to the position of the sensor chip on the wafer. The identifier can, e.g. during the first calibration measurement, e.g. at its end, be stored in a memory of the sensor chip. Alternatively, each sensor chip may already have been provided with an optical or electronic unique identifier in an earlier manufacturing step.

Alternatively, or in addition thereto, part or all of the data from the first calibration measurement, or data derived from the first calibration measurement, such as part of the calibration parameters, can also be stored in the sensor chip directly.

In a next step according to Figure 1, the wafer is transferred to a work station 3 of the production line. In this work station 3, the wafer is further manufactured. In one example, the wafer is covered by a cap substrate in the work station 3 for protecting the sensing elements. This manufacturing step may effect tensions in the wafer. After this manufacturing step, the wafer is returned to the apparatus 2 and a further calibration measurement is run on the wafer. Further calibration data is determined and is stored in the control unit 24 (see Figure 3) of the apparatus, and the control unit 24 calculates final calibration parameters from the first and the further calibration data as described above. Preferably at the present station including the apparatus, the corresponding final calibration parameters are stored in the memory of each sensor chip. Then, the wafer may be forwarded to a different work station 5 where the wafer is separated into the individual sensor chips.

In a different embodiment, the wafer is already separated into the individual sensor chips at work station 3, such that the separate sensor chips are created. These are fed to another calibration apparatus adapted and structured to calibrate separated sensor chips. Such other apparatus may, e.g. be prepared to calibrate individual sensor chips rather than an entire wafer of sensor chips. However, even without an interim separation of the sensor chips, the first and the further calibration measurements may be performed on different apparati.

The unique identifier attributed to each sensor chip as described above can be used after the further calibration step for retrieving the correct first calibration data of each sensor chip in order to calculate the calibration parameters.

The arrows between the various stations in the production line preferably indicate means for transporting the wafer or the sensor chips between the stations. This means may comprise pickers, band conveyors, etc. A production line control unit is referred to by 6 and controls e.g. the conveying of the wafers, etc. In one embodiment, the first calibration data (or data derived therefrom) may be stored in a storage associated with the production line control unit 6.

Figure 2 illustrates a schematic production line according to another embodiment of the present invention. The way of manufacturing and calibrating sensor chips via station 1 to 2 to 3 and back to 2 is identical to the embodiment of Figure 1. However, it is assumed that at work station 3 a first manufacturing step is carried out on the whole wafer, e.g. for adding additional coatings or structures. During this step it is assumed that the wafer may be exposed to stress. Hence the wafer is transferred back to the apparatus 2 for conducting a second calibration measurement. Calibration data resulting from the second calibration measurement (or data derived therefrom) is stored in the storage of the production line as is the first calibration data (or data derived therefrom), e.g. in a storage of the control unit 24, in a storage of a computing device determining the calibration data, or in a storage of the production line control unit.

In a next step, the wafer is forwarded to another work station 4 for having a further manufacturing step performed. In the present example, this further manufacturing step includes the separation of the sensor chips from the wafer, e.g. by dicing. This dicing step may again impact the sensor chips such that a further calibration measurement is performed, this time with another apparatus 2' adapted and structured to calibrate individual sensor chips and not whole wafers. After having determined final calibration parameters from the first, the second and the further calibration measurement, the corresponding final calibration data is stored in the memory of the sensor chips, e.g. during the further calibration measurement in apparatus 2'. The sensor chips are then forwarded to work station 5, where further work may be performed on the sensor chips.

## Claims

1. A method for calibrating a sensor chip, comprising
   performing a first calibration measurement with the sensor chip (9) and determining first calibration data,
   performing a manufacturing step on the sensor chip (9), thereby mechanically and/or chemically impacting a sensing element or an electronic circuitry of the sensor chip (9),
   performing a further calibration measurement with the sensor chip (9) and determining further calibration data,
   determining at least one calibration parameter from the first and the further calibration data,
   storing the at least one calibration parameter in a memory (94) of the sensor chip (9), and
   applying the at least one calibration parameter to a signal sensed by the sensing element (92) of the sensor chip (9) during operation.

2. The method of claim 1,
   wherein the first calibration measurement is performed with the sensor chip (9) yet being an integral part of a wafer (7) to manufacture multiple sensor chips (9) from, and
   wherein the further calibration measurement is performed after the sensor chip (9) is separated from the wafer (7).

3. The method of any one of the preceding claims,
   wherein the manufacturing step includes packaging the sensor chip (9).

4. The method of any one of the preceding claims,
   wherein the manufacturing step includes one or more of:

   - applying a layer to the sensor chip (9),
   - casting a mould compound to the sensor chip (9),
   - attaching contact elements, in particular solder bumps, to the sensor chip (9),
   - applying a membrane to a packaged sensor chip (9),
   - attaching, in particular soldering the sensor chip (9) to a circuit board or a leadframe,
   - separating the sensor chip (9) from a wafer (7).

5. The method of any one of the preceding claims,
   wherein the first calibration data, or data derived from the first calibration data, is at least temporarily stored in a storage of a production line.

6. The method of any one of the preceding claims, in the first calibration measurement, a larger number of measured values is obtained in the first calibration measurement than in the further calibration measurement.

7. The method of any one of the preceding claims, comprising
   performing a second calibration measurement after the manufacturing step with the sensor chip (9) and determining second calibration data,
   performing a further manufacturing step on the sensor chip (9) after having performed the second calibration measurement,
   performing the further calibration measurement with the sensor chip (9) and determining the further calibration data, and
   determining the at least one calibration parameter from the first, the second and the further calibration data.

8. The method of claim 7,
   wherein the second calibration data is at least temporarily stored in a storage of a production line outside said sensor chip.

9. The method of any one of the preceding claims,
   wherein the sensor chip (9) comprises the sensing element (92) sensitive to a substance of a fluid or sensitive to a property of an environment of the sensor chip (9),
   wherein the sensing element (92) is exposed to a fluid with a defined amount of said substance or an environment with a defined magnitude of said property respectively during the first calibration measurement and / or the further calibration measurement, and the second calibration measurement where applicable.

10. The method of any of the preceding claims,
    wherein the sensing element (92) is sensitive to at least one of:

    - temperature;
    - a gas component, in particular humidity;
    - pressure;
    - gas or liquid flow.

11. The method of any of the preceding claims further comprising the steps of
    attributing a unique identifier to said sensor chip (9) during said first calibration step,
    storing said unique identifier together with said first calibration data, or data derived from said first calibration data, in a storage outside said sensor chip,
    using said unique identifier to retrieve said first calibration data, or the data derived from said first calibration data, in order to determine said at least one calibration parameter.

12. The method of claim 11 further comprising the step of storing, in said first calibration measurement, said unique identifier in said sensor chip (9).

13. Production line configured to manufacture a calibrated sensor chip according to the method of any one of the preceding claims, comprising
    a first apparatus (2) configured to perform the first calibration measurement,
    a work station (3) configured to perform the manufacturing step, and
    the first or a further apparatus (2) configured to perform the further calibration measurement, and
    a computing device (24, 6) configured to determine a final calibration data determined from the first, a second and the further calibration data.

**Patentansprüche**

1. Ein Verfahren zum Kalibrieren eines Sensorchips, umfassend
   Durchführen einer ersten Kalibrierungsmessung mit dem Sensorchip (9) und Bestimmen der ersten Kalibrierungsdaten,
   Durchführen eines Herstellungsschritts am Sensorchip (9), wodurch auf ein Sensorelement oder eine elektronische Schaltung des Sensorchips (9) mechanisch und/oder chemisch eingewirkt wird,
   Durchführen einer weiteren Kalibrierungsmessung mit dem Sensorchip (9) und Ermitteln weiterer Kalibrierungsdaten,

Bestimmen mindestens eines Kalibrierungsparameters aus den ersten und den weiteren Kalibrierungsdaten, Speichern des mindestens einen Kalibrierungsparameters in einem Speicher (94) des Sensorchips (9), und Anwenden des mindestens einen Kalibrierungspararameters auf ein Signal, das vom Sensorelement (92) des Sensorchips (9) während des Betriebs erfasst wird.

2. Das Verfahren nach Anspruch 1,
wobei die erste Kalibrierungsmessung durchgeführt wird während der Sensorchip (9) noch ein integraler Teil eines Wafers (7) ist, um mehrere Sensorchips (9) herzustellen, und
wobei die weitere Kalibrierungsmessung nach der Trennung des Sensorchips (9) vom Wafer (7) durchgeführt wird.

3. Das Verfahren nach einem der vorangehenden Ansprüche,
wobei der Herstellungsschritt das Verpacken des Sensorchips (9) umfasst.

4. Das Verfahren nach einem der vorangehenden Ansprüche,
wobei der Herstellungsschritt einen oder mehrere der folgenden Schritte umfasst:

- Aufbringen einer Schicht auf den Sensorchip (9),
- Giessen einer Formmasse auf den Sensorchip (9),
- Anbringen von Kontaktelementen, insbesondere Löthöckern, auf dem Sensorchip (9),
- Aufbringen einer Membran auf einen verpackten Sensorchip (9),
- Anbringen, insbesondere Verlöten des Sensorchips (9) auf einer Leiterplatte oder einem Leadframe,
- Trennen des Sensorchips (9) vom Wafer (7).

5. Das Verfahren nach einem der vorangehenden Ansprüche,
wobei die ersten Kalibrierungsdaten oder von den ersten Kalibrierungsdaten abgeleitete Daten zumindest vorübergehend in einem Speicher einer Produktionslinie gespeichert werden.

6. Das Verfahren nach einem der vorangehenden Ansprüche, wobei bei der ersten Kalibrierungsmessung eine grössere Anzahl von Messwerten als bei der weiteren Kalibrierungsmessung erhalten wird.

7. Das Verfahren nach einem der vorangehenden Ansprüche, umfassend
Durchführen einer zweiten Kalibrierungsmessung nach dem Herstellungsschritt mit dem Sensorchip (9) und Ermitteln der zweiten Kalibrierungsdaten,
Durchführen eines weiteren Fertigungsschritts am Sensorchip (9) nach Durchführung der zweiten Kalibrierungsmessung,
Durchführen der weiteren Kalibrierungsmessung mit dem Sensorchip (9) und Ermitteln der weiteren Kalibrierungsdaten, und
Bestimmen des mindestens einen Kalibrierungsparameters aus den ersten, zweiten und weiteren Kalibrierungsdaten.

8. Das Verfahren nach Anspruch 7,
wobei die zweiten Kalibrierungsdaten zumindest vorübergehend in einem Speicher einer Produktionslinie ausserhalb des Sensorchips gespeichert werden.

9. Das Verfahren nach einem der vorangehenden Ansprüche,
wobei der Sensorchip (9) das Sensorelement (92) umfasst, das für eine Substanz eines Fluids oder für eine Eigenschaft einer Umgebung des Sensorchips (9) empfindlich ist,
wobei das Sensorelement (92) während der ersten Kalibrierungsmessung und/oder der weiteren Kalibrierungsmessung und gegebenenfalls der zweiten Kalibrierungsmessung einem Fluid mit einer definierten Menge der Substanz oder einer Umgebung mit einer definierten Grösse der Eigenschaft ausgesetzt ist.

10. Das Verfahren nach einem der vorangehenden Ansprüche,
wobei das Sensorelement (92) für mindestens eine der nachfolgenden Eigenschaften empfindlich ist:

- Temperatur;
- einer Gaskomponente, insbesondere Feuchtigkeit;
- Druck;
- Gas- oder Flüssigkeitsstrom.

**11.** Das Verfahren nach einem der vorangehenden Ansprüche, weiter umfassend die folgenden Schritte:

Zuweisen dem Sensorchip (9) während des ersten Kalibrierungsschritts eine eindeutige Kennung,
Speichern der eindeutigen Kennung zusammen mit den ersten Kalibrierungsdaten oder von den ersten Kalibrierungsdaten abgeleiteten Daten in einem Speicher ausserhalb des Sensorchips,
Verwenden der eindeutigen Kennung zum Abrufen der ersten Kalibrierungsdaten oder der von den ersten Kalibrierungsdaten abgeleiteten Daten, um den mindestens einen Kalibrierungsparameter zu bestimmen.

**12.** Das Verfahren nach Anspruch 11, weiter umfassend den Schritt des Speicherns der eindeutigen Kennung im Sensorchip bei der ersten Kalibrierungsmessung (9).

**13.** Produktionslinie, konfiguriert zur Herstellung eines kalibrierten Sensorchips gemäss dem Verfahren nach einem der vorangehenden Ansprüche, umfassend
eine erste Vorrichtung (2), konfiguriert zur Durchführung der ersten Kalibrierungsmessung,
eine Arbeitsstation (3), konfiguriert zur Durchführung des Herstellungsschrittes, und
die erste oder eine weitere Vorrichtung (2), konfiguriert zur Durchführung der weiteren Kalibriermessung, und
eine Recheneinrichtung (24, 6), konfiguriert zur Bestimmung von endgültigen Kalibrierungsdaten, die aus den ersten, den zweiten und den weiteren Kalibrierungsdaten bestimmt wird.

**Revendications**

**1.** Un procédé de calibration d'une puce de capteur, comprenant
effectuer une première mesure de calibration avec la puce de capteur (9) et déterminer les premières données de calibration,
effectuer une étape de fabrication sur la puce de capteur (9), ce qui a un impact mécanique et/ou chimique sur un élément de détection ou un circuit électronique de la puce de capteur (9),
effectuer une autre mesure de calibration avec la puce de capteur (9) et déterminer d'autres données de calibration,
déterminer au moins un paramètre de calibration à partir de la première et des autres données de calibration,
stocker l'au moins un paramètre de calibration dans une mémoire (94) de la puce de capteur (9), et
appliquer l'au moins un paramètre de calibration à un signal détecté par l'élément de détection (92) de la puce de capteur (9) pendant le fonctionnement.

**2.** Le procédé selon la revendication 1,
dans lequel la première mesure de calibration est effectuée avec la puce de capteur (9) qui est pourtant une partie intégrante d'une plaquette (7) pour fabriquer plusieurs puces capteur (9), et
dans lequel la mesure de calibration supplémentaire est effectuée après que la puce de capteur (9) a été séparée de la plaquette (7).

**3.** Le procédé selon l'une quelconque des revendications précédentes,
dans lequel l'étape de fabrication comprend l'emballage de la puce de capteur (9).

**4.** Le procédé selon l'une quelconque des revendications précédentes,
dans lequel l'étape de fabrication comprend une ou plusieurs :

- appliquer une couche sur la puce de capteur (9),
- couler un composé de moulage sur la puce de capteur (9),
- fixer d'éléments de contact, en particulier de bosses de soudure, sur la puce de capteur (9),
- appliquer une membrane sur une puce de capteur emballée (9),
- fixer, en particulier souder la puce de capteur (9) à une carte de circuit imprimé ou à une grille de connexion,
- séparer la puce de capteur (9) de la plaquette (7).

**5.** Le procédé selon l'une quelconque des revendications précédentes,
dans lequel les premières données de calibration, ou les données dérivées des premières données de calibration, sont au moins temporairement stockées dans une mémoire d'une chaîne de production.

**6.** Le procédé selon l'une quelconque des revendications précédentes, dans la première mesure de calibration, un plus grand nombre de valeurs mesurées est obtenu dans la première mesure de calibration que dans la mesure de calibration ultérieure.

**7.** Le procédé selon l'une quelconque des revendications précédentes, comprenant
effectuer une deuxième mesure de calibration après l'étape de fabrication avec la puce de capteur (9) et déterminer des données du deuxième calibration,
effectuer une nouvelle étape de fabrication sur la puce de capteur (9) après avoir effectué la deuxième mesure de calibration,
effectuer la mesure de calibration supplémentaire avec la puce de capteur (9) et déterminer les données de calibration supplémentaires, et
déterminer l'au moins un paramètre de calibration à partir de la première, de la deuxième et des autres données de calibration.

**8.** Le procédé selon la revendication 7,
dans lequel les deuxièmes données de calibration sont au moins temporairement stockées dans un stockage d'une chaîne de production à l'extérieur de ladite puce de capteur.

**9.** Le procédé selon l'une quelconque des revendications précédentes,
dans lequel la puce de capteur (9) comprend l'élément de détection (92) sensible à une substance d'un fluide ou sensible à une propriété d'un environnement de la puce de capteur (9),
dans lequel l'élément de détection (92) est exposé à un fluide contenant une quantité définie de ladite substance ou à un environnement ayant une grandeur définie de ladite propriété respectivement pendant la première mesure de calibration et/ou la mesure de calibration ultérieure, et la deuxième mesure de calibration le cas échéant.

**10.** Le procédé selon l'une des revendications précédentes,
dans lequel l'élément de détection (92) est sensible à au moins un des éléments suivants

- la température ;
- un composant gazeux, en particulier l'humidité ;
- la pression ;
- le débit de gaz ou de liquide.

**11.** Le procédé selon l'une quelconque des revendications précédentes comprenant en outre les étapes suivantes
attribuer un identifiant unique à ladite puce de capteur (9) au cours de ladite première étape de calibration,
stocker ledit identifiant unique avec lesdites premières données de calibration, ou des données dérivées desdites premières données de calibration, dans un stockage à l'extérieur de ladite puce de capteur,
utiliser ledit identificateur unique pour récupérer lesdites premières données de calibration, ou les données dérivées desdites premières données de calibration, afin de déterminer ledit au moins un paramètre de calibration.

**12.** Le procédé selon la revendication 11 comprenant en outre l'étape de stocker, dans ladite première mesure de calibration, ledit identificateur unique dans ladite puce de capteur (9).

**13.** Ligne de production configurée pour la fabrication d'une puce de capteur calibrée selon le procédé selon l'une quelconque des revendications précédentes, comprenant
un premier appareil (2) configurée pour effectuer la première mesure de calibration,
un poste de travail (3) configurée pour exécuter l'étape de fabrication, et
le premier ou un autre appareil (2) configurée pour effectuer la mesure de calibration ultérieure, et
un dispositif de calcul (24, 6) configurée pour déterminer une donnée de calibration finale déterminée à partir de la première, de la deuxième et des autres données de calibration.

FIG. 1

FIG. 2

24

7

221

22

z y

21 x

23

FIG. 3

93      92      94

9

91

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0142776 A
- EP 1628132 A1

- EP 1628132 A

**Non-patent literature cited in the description**

- **MICHAEL DEEDS et al.** Carrier-level Packaging and Reliability of a MEMS-Based Safety and Arming Device. *MRS Proceedings,* 31 December 2001, vol. 682E, 1-12